(19) Europäisches Patentamt
European Patent Office
Office européen des brevets

(11) Veröffentlichungsnummer: **0 023 305**
**B1**

(12) # EUROPÄISCHE PATENTSCHRIFT

(45) Veröffentlichungstag der Patentschrift: **02.03.83**

(51) Int. Cl.³: **C 07 D 295/18**, C 07 D 241/04, C 07 D 243/08, A 01 N 25/32, C 07 D 233/02

(21) Anmeldenummer: **80104058.5**

(22) Anmeldetag: **14.07.80**

(54) **Verwendung von N,N'-Bis-(halogenacyl)-diaza-cycloalkanen zum Schutz von Kulturpflanzen vor Schädigungen durch Herbizide.**

(30) Priorität: **26.07.79 DE 2930452**

(43) Veröffentlichungstag der Anmeldung: **04.02.81 Patentblatt 81/5**

(45) Bekanntmachung des Hinweises auf die Patenterteilung: **02.03.83 Patentblatt 83/9**

(84) Benannte Vertragsstaaten: **BE CH DE FR GB IT LI NL**

(56) Entgegenhaltungen:
**DE-A-2 328 870**
**US-A-3 090 338**

**Rocz chem. 47(6), 1277–1280 (1973), ibid 47, 1937–1942 (1973), ibid 30, 229–234 (1969)**
**JOURNAL OF AGRICULTURAL AND FOOD CHEMISTRY, Band 26, Nr. 1, 1978, Washington, D.C., US, G.R. STEPHENSON et al.: »Structure activity relationships for S-ethyl N,N-Dipropylthiocarbamate (EPTC) Antidotes in Corn«, Seiten 137–140**
**JOURNAL OF MEDICINAL CHEMISTRY, Band 11, Mai 1968, Washington, D.C., US, S. GROSZKOWSKI et al.: »Cytostatic Bis(halcacyl) derivatives of piperazine and 2-methylpiperazine« Seiten 821–2**

(73) Patentinhaber: **BAYER AG, Zentralbereich Patente, Marken und Lizenzen, D-5090 Leverkusen 1, Bayerwerk (DE)**

(72) Erfinder: **Riebel, Hans-Jochem, Dr., In der Beek 92, D-5600 Wuppertal 1 (DE)**
Erfinder: **Eue, Ludwig, Dr., Paul-Klee-Strasse 36, D-5090 Leverkusen 1 (DE)**
Erfinder: **Faust, Wilfried, Dr., Eifgenstrasse 16, D-5068 Odenthal 3 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

Verwendung von N,N'-Bis-(halogenacyl)-diaza-cycloalkanen zum Schutz von Kulturpflanzen vor Schädigungen durch Herbizide

Die vorliegende Erfindung betrifft die Verwendung von teilweise bekannten N,N'-Bis-(halogenacyl)-diaza-cycloalkanen als Gegenmittel zum Schutz von Kulturpflanzen vor Schädigungen durch herbizid wirksame Acetanilide. Ferner betrifft die vorliegende Erfindung neue Wirkstoffkombinationen, die aus teilweise bekannten N,N'-Bis-(halogenacyl)-diaza-cycloalkanen und bestimmten herbizid wirksamen Acetaniliden bestehen und besonders gute selektive herbizide Eigenschaften besitzen.

Unter »Gegenmittel« (»Safener«, »Antidote«) sind im vorliegenden Zusammenhang Stoffe zu verstehen, welche befähigt sind, schädigende Wirkungen von Herbiziden auf Kulturpflanzen spezifisch zu antagonisieren, d. h. die Kulturpflanzen zu schützen, ohne dabei die Herbizidwirkung auf die zu bekämpfenden Unkräuter merklich zu beeinflussen.

Es ist bekannt, daß bestimmte Acetanilide beim Einsatz zur Unkrautbekämpfung in Mais und anderen Kulturen mehr oder weniger starke Schäden an den Kulturpflanzen hervorrufen. Weiterhin ist bekannt, daß Verbindungen wie z. B, N-Dichloracetyl-2-ethyl-piperidin und N-Dichloracetyl-cis, trans-decahydrochinolin geeignet sind, um Schädigungen durch Thiolcarbamate oder Acetanilide an Kulturpflanzen zu vermindern (vgl. DE-OS 2 218 097). Die Wirksamkeit dieser Stoffe als Gegenmittel ist jedoch nicht immer ganz befriedigend.

Weiterhin sind bereits zahlreiche N,N'-Bis(halogenacyl)-diaza-cycloalkane beschrieben worden (vgl. US-PS 3 898 338; J. Med. Chem. 11, 621—622 (1969); Rocz. Chem. 47, 1277 bis 1280 (1973); Rocz. Chem, 1937 bis 1942 (1973); Rocz. Chem. 38, 229 bis 235 (1964) und DE-OS 2 328 870). Von einigen dieser Stoffe ist bekannt, daß sie cytostatische bzw. anthelmintische oder auch anti-tumorale Wirksamkeit aufweisen. So lassen sich zum Beispiel 1,4-Bis-(halogenacyl)-piperazine, wie 1,4-Bis-($\alpha$-Chlorpropionyl)-piperazin oder 1,4-Bis-($\alpha$-Brom-propionyl)-piperazin als Cytostatika verwenden (vgl. J. Med. Chem. 11, (1968), 621—622). Darüber hinaus ist der Literatur zu entnehmen, daß bestimmte 1,4-Bis-(halogenacyl)-piperazine als Zwischenprodukte zur Herstellung von Stoffen mit schleimauflösender Wirkung eingesetzt werden können (vgl. US-PS 3 898 338).

Schließlich wird in dem Artikel in J. Arg. Food Chem. 26, 137—140 (1978) eine vergleichende Übersicht über die antagonistischen Eigenschaften zahlreicher Amide auf Thiolcarbamate gegeben; Extrapolationen auf das Verhalten solcher Amide bei herbizid wirksamen Acetaniliden lassen sich jedoch nicht machen.

Es wurde jetzt gefunden, daß die teilweise bekannten N,N'-Bis-(halogenacyl)-diazacycloalkane der Formel

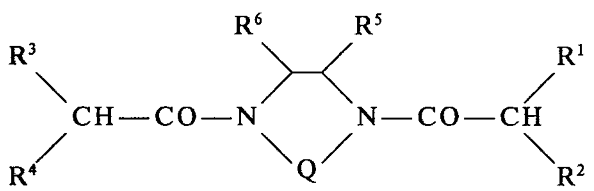

(I)

in welcher

$R^1$ für geradkettiges oder verzweigtes Alkyl mit 1 bis 4 Kohlenstoffatomen, Chlor oder Brom steht,
$R^2$ für Chlor oder Brom steht,
$R^3$ für geradkettiges oder verzweigtes Alkyl mit 1 bis 4 Kohlenstoffatomen, Chlor oder Brom steht,
$R^4$ für Chlor oder Brom steht,
$R^5$ für Wasserstoff oder Alkyl mit 1 oder 2 Kohlenstoffatomen steht,
$R^6$ für Wasserstoff oder Alkyl mit 1 oder 2 Kohlenstoffatomen steht und
Q für eine gegebenenfalls ein- oder mehrfach durch Methyl oder Ethyl substituierte Alkylenkette mit 2 oder 3 Kohlenstoffatomen in der Alkylenkette steht,

hervorragend geeignet sind, um Kulturpflanzen vor Schädigungen durch herbizid wirksame Acetanilide zu schützen.

Weiterhin wurde gefunden, daß die neuen Wirkstoffkombinationen bestehend aus einem N,N'-Bis-(halogenacyl)-diaza-cycloalkan der Formel (I) und mindestens einem herbizid wirksamen Acetanilid hervorragend geeignet sind zur selektiven Unkrautbekämpfung in Nutzpflanzenkulturen.

Überraschenderweise werden Herbizidschädigungen durch Acetanilide an Kulturpflanzen bei Mitverwendung von N,N'-Bis-(halogenacyl)-diaza-cycloalkanen der Formel (I) besser unterdrückt als beim Einsatz der bekannten Verbindungen N-Dichloracetyl-2-ethyl-piperidin und N-Dichloracetyl-cis, trans-decahydrochinolin, welches chemisch ähnliche Stoffe gleicher Wirkungsart sind. Im übrigen war nicht zu erwarten, daß die erfindungsgemäßen Wirkstoffkombinationen bessere selektive herbizide Eigenschaften besitzen als Wirkstoffkombinationen, welche aus mindestens einem herbizid wirksamen Acetanilid und dem als Gegenmittel bekannten N-Dichloracetyl-2-ethyl-piperidin oder dem ebenfalls als Gegenmittel bekannten N-Dichloracetyl-cis, trans-decahydrochinolin bestehen.

Die erfindungsgemäß verwendbaren N,N'-(halogenacyl)-diaza-cycloalkane der Formel (I) sind teilweise bekannt (vgl. US-PS 3 898 338; J. Med. Chem. 11, 621–622 (1968); Rocz. Chem. 47, 1277–1280 (1973); Rocz. Chem. 38, 229–234 (1964) und Rocz. Chem. 47 (1973), (1937–1942). Die bisher noch nicht beschriebenen Stoffe der Formel (I) lassen sich in einfacher Weise nach im Prinzip bekannten Verfahren herstellen. So erhält man N,N'-Bis-(halogenacyl)-diaza-cycloalkane der Formel (I), indem man

a) Diaza-cycloalkane der Formel

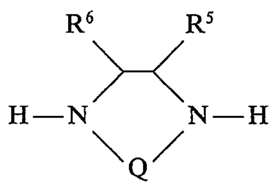

(II)

in welcher

$R^5$, $R^6$ und Q die oben angegebene Bedeutung haben,

mit Alkanoylchloriden der Formel

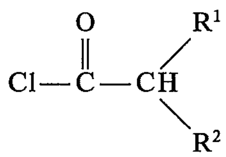

(IIIa)

in welcher

$R^1$ und $R^2$ die oben angegebene Bedeutung haben,

gegebenenfalls in Gegenwart eines Verdünnungsmittels sowie gegebenenfalls in Gegenwart eines Säurebindemittels umsetzt,

oder

b) N-Halogenacyl-diaza-cycloalkane der Formel

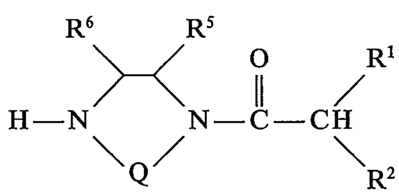

(IV)

in welcher

$R^1$, $R^2$, $R^5$, $R^6$ und Q die oben angegebene Bedeutung haben,

mit Alkanoylchloriden der Formel

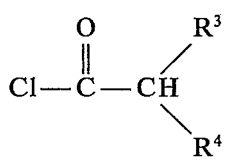

(IIIb)

in welcher
$R^3$ und $R^4$ die oben angegebene Bedeutung haben,
gegebenenfalls in Gegenwart eines Verdünnungsmittels sowie gegebenenfalls in Gegenwart eines Säurebindemittels umsetzt.

Die bei der Durchführung des Verfahrens (a) als Ausgangsstoffe benötigten Diaza-cycloalkane der Formel (II) sind bekannt oder lassen sich nach im Prinzip bekannten Verfahren herstellen.

Die bei der Durchführung des Verfahrens (b) als Ausgangsstoffe benötigten N-Halogenacyldiaza-cycloalkane der Formel (IV) sind bekannt oder lassen sich nach im Prinzip bekannten Verfahren in einfacher Weise herstellen. So können die betreffenden Stoffe zum Beispiel erhalten werden, wenn man bei der Umsetzung nach dem beschriebenen Verfahren (a) das Diazacycloalkan der Formel (II) mit einer für eine einfache Halogenacylierung berechneten stöchiometrischen Menge an Alkanoylchlorid der Formel (IIIa) umsetzt.

Die bei den Verfahren (a) und (b) weiterhin als Ausgangsstoffe benötigten Alkanoylchloride der Formeln (IIIa) und (IIIb) sind ebenfalls bekannt oder lassen sich nach im Prinzip bekannten Verfahren in einfacher Weise herstellen (vgl. DE-OS 2 218 097).

Die Verfahren (a) und (b) zur Herstellung der erfindungsgemäß verwendbaren N,N'-Bis-(halogenacyl)-diaza-cycloalkane der Formel (I) werden vorzugsweise in Gegenwart eines Verdünnungsmittels durchgeführt. Als Verdünnungsmittel können hierbei Wasser sowie inerte organische Lösungsmittel verwendet werden. Hierzu gehören vorzugsweise Ketone, wie Diethylketon und Methylisobutylketon; Nitrile, wie Propionitril und Acetonitril; Ether, wie Tetrahydrofuran und Dioxan, aliphatische und aromatische Kohlenwasserstoffe, wie Petrolether, Benzol, Toluol oder Xylol, halogenierte Kohlenwasserstoffe, wie Methylenchlorid, Tetrachlorkohlenstoff, Chloroform oder Chlorbenzol; Ester, wie Essigester; und Formamide, wie insbesondere Dimethylformamid.

Als Säurebindemittel kommen sowohl bei der Herstellung der erfindungsgemäß verwendbaren N,N'-Bis-(halogenacyl)-diaza-cycloalkane der Formel (I) nach dem Verfahren (a) als auch nach dem Verfahren (b) alle üblichen Säureakzeptoren in Betracht. Hierzu gehören vorzugsweise Alkalihydroxide, wie Natriumhydroxid und Kaliumhydroxid, ferner Alkalicarbonate, wie Natriumcarbonat, Kaliumcarbonat und Natriumhydrogencarbonat, und weiterhin niedere tertiäre Amine, wie Triethylamin, Dimethylbenzylamin, Pyridin und Diazabicyclooctan. Es kann jedoch auch im Überschuß eingesetztes Diaza-cycloalkan der Formel (II) (Verfahren a) bzw. N-(Halogenacyl)-diaza-cycloalkan der Formel (IV) (Verfahren b) gleichzeitig als Säurebindemittel fungieren. In diesen Fällen erübrigt sich die gesonderte Zugabe eines zusätzlichen Säurebindemittels.

Die Reaktionstemperaturen können sowohl bei dem Verfahren (a) als auch bei dem Verfahren (b) innerhalb eines größeren Bereiches variiert werden. Im allgemeinen arbeitet man jeweils bei Temperaturen zwischen 0 und 100°C, vorzugsweise zwischen 10 und 80°C.

Bei der Durchführung des Verfahrens (a) setzt man auf 1 Mol an Diaza-cycloalkan der Formel (II) vorzugsweise 2 bis 3 Mol an Alkanoylchlorid der Formel (IIIa) sowie gegebenenfalls 1 Mol an Säurebindemittel ein. Die Isolierung der Reaktionsprodukte erfolgt nach üblichen Methoden. Im allgemeinen verfährt man in der Weise, daß man den gegebenenfalls nach vorherigem Versetzen des Reaktionsgemisches mit Wasser anfallenden Niederschlag absaugt, wäscht und umkristallisiert.

Bei der Durchführung des Verfahrens (b) setzt man auf 1 Mol an N-Halogenacyl-diaza-cycloalkan der Formel (IV) vorzugsweise 1 Mol an Alkanoylchlorid der Formel (IIIb) sowie gegebenenfalls 1 Mol an Säurebindemittel ein. Die Isolierung der Reaktionsprodukte erfolgt nach üblichen Methoden. Im allgemeinen arbeitet man so, wie es bereits im Zusammenhang mit dem Verfahren (a) beschrieben wurde.

Die erfindungsgemäß verwendbaren N,N'-Bis-(halogenacyl)-cycloalkane der Formel (I) eignen sich, — wie bereits erwähnt —, zum Schutz von Kulturpflanzen vor Schädigungen durch herbizid wirksame Acetanilide, ohne deren Unkrautwirkung merklich zu beeinflussen.

Vorzugsweise können die N,N'-Bis-(halogenacyl)-diaza-cycloalkane der Formel (I) zum Schutz von Kulturpflanzen vor Schädigungen durch herbizide wirksame Acetanilide der Formel

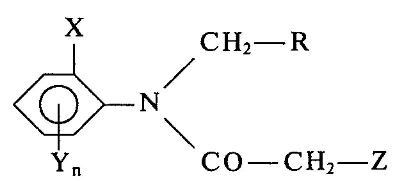

(V)

in welcher

R für einen gegebenenfalls substituierten N-haltigen heterocyclischen Rest steht,
X und Y gleich oder verschieden sind und für Alkyl stehen,
Z für Halogen steht und
n für 0, 1 oder 2 steht,

beziehungsweise der Formel

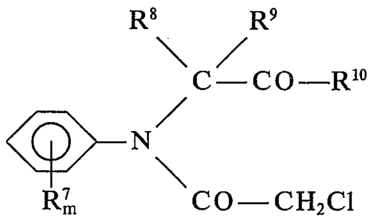

(VI)

in welcher

$R^7$ für Alkyl, Halogen, Halogenalkyl, Alkylthio, Alkylsulfonyl, Aminosulfonyl, Cyano oder Nitro steht,
$R^8$ und $R^9$ gleich oder verschieden sind und für Wasserstoff, Alkyl, Halogen, Halogenalkyl oder gegebenenfalls substituiertes Phenyl stehen,
$R^{10}$ für Alkyl oder gegebenenfalls substituiertes Phenyl steht und
m für ganze Zahlen von 0 bis 5 steht,

beziehungsweise der Formel

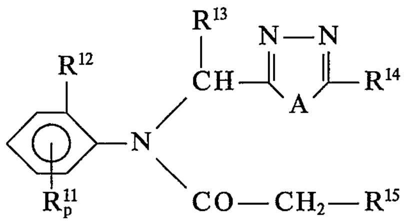

(VII)

in welcher

A für Sauerstoff, Schwefel oder die Gruppierung $>NR^{16}$ steht,
$R^{13}$ für Wasserstoff oder Alkyl steht,
$R^{14}$ für Wasserstoff, Alkyl, Halogenalkyl, Alkenyl, Alkinyl, Cycloalkyl, Halogen, gegebenenfalls substituiertes Aryl und Aralkyl oder die Gruppierungen $-OR^{17}$, $-SR^{17}$ und $-NR^{16}R^{17}$ steht,
$R^{16}$ für Wasserstoff, Alkyl, oder gegebenenfalls substituiertes Aryl steht,
$R^{17}$ für Wasserstoff, Alkyl, Halogenalkyl, Alkenyl, Alkinyl, Cycloalkyl oder gegebenenfalls substituiertes Aralkyl steht,
$R^{11}$ für Alkyl steht,
$R^{12}$ für Alkyl oder Halogen steht,
$R^{15}$ für Halogen steht und
p für die Zahlen 0, 1 oder 2 steht,

beziehungsweise der Formel

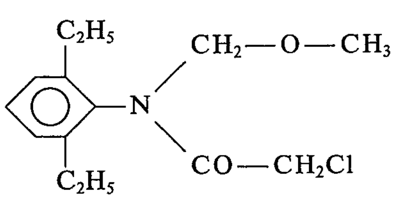

(VIII)

– oder der Formel

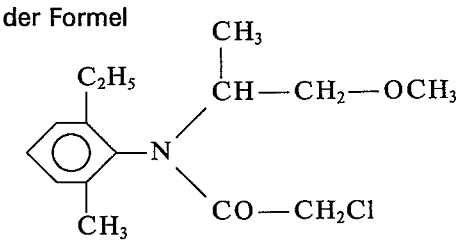

(IX)

verwendet werden.

In der Formel (V) steht R vorzugsweise für die gegebenenfalls substituierten Azolylreste Pyrazol-1-yl, Imidazol-1-yl, 1,2,4-Triazol-1-yl; 1,2,3-Triazol-1-yl; 1,3,4-Triazol-1-yl und 1,2,3,4-Tetrazol-1-yl

sowie für gegebenenfalls substituiertes Pyrrol-1-yl. Als Substituenten kommen vorzugsweise in Frage: Halogen, insbesondere Fluor, Chlor und Brom, sowie Alkyl mit 1 bis 4 Kohlenstoffatomen. X und Y sind gleich oder verschieden und stehen vorzugsweise für geradkettiges oder verzweigtes Alkyl mit 1 bis 4 Kohlenstoffatomen. Z steht vorzugsweise für die Halogene Chlor und Brom und der Index n steht für 0,1 oder 2.

Als Beispiele für Acetanilide der Formel (V) seien im einzelnen genannt:

2-Methyl-6-ethyl-N-(pyrazol-1-yl-methyl)-chloracetanilid
2,6-Diethyl-N-(pyrazol-1-yl-methyl)-chloracetanilid
2,6-Diethyl-N-(1,2,4-triazol-1-yl-methyl)-chloracetanilid
2,6-Dimethyl-N-(1,2,4-triazol-1-yl-methyl)-chloracetanilid
2-Methyl-N-(pyrazol-1-yl-methyl)-chloracetanilid
2,5-Dimethyl-N-(pyrazol-1-yl-methyl)-chloracetanilid
2,3-Dimethyl-N-(pyrazol-1-yl-methyl)-chloracetanilid
2,6-Diethyl-N-[(3,5-dimethyl-pyrazol-1-yl)-methyl]-chloracetanilid
2,6-Diethyl-N-[(3-chlor-1,2,4-triazolyl)-methyl]-chloracetanilid
2-Methyl-6-ethyl-N-[(3,5-dimethyl-pyrazol-1-yl)-methyl]-chloracetanilid
2-tert.-Butyl-N-(pyrazol-1-yl-methyl)-chloracetanilid
2-Methyl-6-ethyl-N-[(3-brom-5-methyl-pyrazol-1-yl)-methyl]-chloracetanilid
2-Methyl-6-ethyl-N-[(3-chlor-1,2,4-triazolyl)-methyl]-chloracetanilid
2,6-Diethyl-N-[(4-chlor-pyrazol-1-yl)-methyl]-chloracetanilid

Weitere bevorzugt in Frage kommende Acetanilide der Formel (V) sind in der nachfolgenden Tabelle 1 aufgeführt.

Tabelle 1

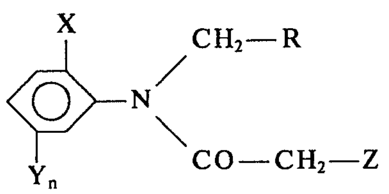

(V)

| Bsp. Nr. | X | Yn | Z | R |
|---|---|---|---|---|
| V-1 | $C_2H_5$ | 6-$C_2H_5$ | Cl | Pyrazol-1-yl |
| V-2 | $C_2H_5$ | 6-$C_2H_5$ | Cl | 1,2,4-Triazol-1-yl |
| V-3 | i-$C_3H_7$ | 6-i-$C_3H_7$ | Cl | Pyrazol-1-yl |
| V-4 | $CH_3$ | 6-$C_2H_5$ | Cl | 1,2,4-Triazol-1-yl |
| V-5 | $CH_3$ | 6-$C_2H_5$ | Cl | Pyrazol-1-yl |
| V-6 | $C_2H_5$ | 4,6-$(CH_3)_2$ | Cl | Pyrazol-1-yl |
| V-7 | $CH_3$ | 4,6-$(CH_3)_2$ | Cl | Pyrazol-1-yl |
| V-8 | $C_2H_5$ | 4-$CH_3$, 6-$C_2H_5$ | Cl | Pyrazol-1-yl |
| V-9 | i-$C_3H_7$ | 6-i-$C_3H_7$ | Cl | 1,3,4-Triazol-1-yl |
| V-10 | i-$C_3H_7$ | 6-i-$C_3H_7$ | Cl | 1,2,4-Triazol-1-yl |
| V-11 | $C_2H_5$ | 6-$C_2H_5$ | Cl | Pyrrol-1-yl |
| V-12 | i-$C_3H_7$ | – | Cl | 1,2,4-Triazol-1-yl |
| V-13 | $CH_3$ | 6-$C_2H_5$ | Cl | 1,2,3,4-Tetrazol-1-yl |

Tabelle 1 (Fortsetzung)

| Bsp. Nr. | X | Yn | Z | R |
|---|---|---|---|---|
| V-14 | i-$C_3H_7$ | – | Cl | Pyrazol-1-yl |
| V-15 | $C_2H_5$ | – | Cl | 1,2,4-Triazol-1-yl |
| V-16 | $CH_3$ | 6-$CH_3$ | Cl | Pyrazol-1-yl |
| V-17 | $CH_3$ | 6-$CH_3$ | Cl | 1,2,4-Triazol-1-yl |
| V-18 | $CH_3$ | 5-$CH_3$ | Cl | 1,2,4-Triazol-1-yl |
| V-19 | $CH_3$ | – | Cl | Pyrazol-1-yl |
| V-20 | $CH_3$ | – | Cl | 1,2,4-Triazol-1-yl |
| V-21 | $CH_3$ | 5-$CH_3$ | Cl | Pyrazol-1-yl |
| V-22 | $CH_3$ | 3-$CH_3$ | Cl | 1,2,4-Triazol-1-yl |
| V-23 | $CH_3$ | 3-$CH_3$ | Cl | Pyrazol-1-yl |
| V-24 | $C_2H_5$ | 6-$C_2H_5$ | Cl | 3,5-Dimethylpyrazol-1-yl |
| V-25 | $C_2H_5$ | 6-$C_2H_5$ | Cl | Brom-methylpyrazol |
| V-26 | $C_2H_5$ | 6-$C_2H_5$ | Cl | 3-Chlor-1,2,4-triazol-1-yl |
| V-27 | $CH_3$ | 6-$C_2H_5$ | Cl | 3,5-Dimethylpyrazol-1-yl |
| V-28 | $C_2H_5$ | 6-$C_2H_5$ | Cl | 3-Methylpyrazol-1-yl |
| V-29 | $C_2H_5$ | 6-$CH_3$ | Cl | 3-Methylpyrazol-1-yl |
| V-30 | $C(CH_3)_3$ | – | Cl | Pyrazol-1-yl |
| V-31 | $C(CH_5)_3$ | – | Cl | 1,2,4-Triazol-1-yl |
| V-32 | $C_2H_5$ | 6-$CH_3$ | Cl | Brom-methylpyrazolyl |
| V-33 | $CH_5$ | 6-$C_2H_5$ | Cl | 4-Chlorpyrazol-1-yl |
| V-34 | $CH_3$ | 6-$C_2H_5$ | Cl | 3-Chlor-1,2,4-triazol-1-yl |
| V-35 | $C_2H_5$ | 6-$CH_3$ | Cl | 2,4,5-Trichlor-imidazol-1-yl |
| V-36 | $C_2H_5$ | 6-$C_2H_5$ | Cl | 4-Chlor-pyrazol-1-yl |
| V-37 | $C_2H_5$ | 6-$C_2H_5$ | Cl | 1,2,3,4-Tetrazol-1-yl |
| V-38 | $C_2H_5$ | 6-$C_2H_5$ | Br | Pyrazol-1-yl |
| V-39 | $CH_3$ | 6-$C_2H_5$ | Br | Pyrazol-1-yl |
| V-40 | $C_2H_5$ | 6-$C_2H_5$ | Cl | Imidazol-1-yl |
| V-41 | $C_2H_5$ | 6-$C_2H_5$ | Br | 1,2,4-Triazol-1-yl |
| V-42 | $CH_3$ | 6-$C_2H_5$ | Br | 1,2,4-Triazol-1-yl |

Die Acetanilide der Formel (V) und deren herbizide Wirksamkeit sind bereits bekannt (vgl. DE-OS 2 648 008 und DE-OS 2 704 281).

In der Formel (VI) steht $R^7$ vorzugsweise für geradkettiges oder verzweigtes Alkyl mit 1 bis 6, insbesondere 1 bis 4 Kohlenstoffatomen, Halogen, insbesondere Fluor, Chlor und Brom, Halogenalkyl mit bis zu 3 Kohlenstoff- und bis zu 5 gleichen oder verschiedenen Halogenatomen, wobei als Halogene insbesondere Fluor und Chlor stehen, beispielhaft sei Trifluormethyl genannt; ferner vorzugsweise für Alkylthio und Alkylsulfonyl mit 1 bis 4 Kohlenstoffatomen im Alkylteil sowie für Aminosulfonyl, Cyano und Nitro. $R^8$ und $R^9$ sind gleich oder verschieden und stehen vorzugsweise für Wasserstoff, geradkettiges oder verzweigtes Alkyl mit 1 bis 4 Kohlenstoffatomen, Halogen, insbeondere Fluor, Chlor und Brom, Halogenalkyl mit bis zu 3 Kohlenstoff- und bis zu 5 gleichen oder verschiedenen Halogenatomen, wobei als Halogene insbesondere Fluor und Chlor stehen sowie vorzugsweise für gegebenenfalls einfach oder mehrfach substituiertes Phenyl, wobei als Substituenten vorzugsweise die für $R^7$ genannten Reste in Frage kommen. $R^{10}$ steht vorzugsweise für geradkettiges oder verzweigtes Alkyl mit 1 bis 6, insbesondere 1 bis 4 Kohlenstoffatomen sowie für gegebenenfalls einfach oder mehrfach substituiertes Phenyl, wobei als Substituenten vorzugsweise in Frage kommen: Alkyl mit 1 bis 4 Kohlenstoffatomen, Halogen, insbesondere Fluor, Chlor und Brom, Halogenalkyl mit bis zu 3 Kohlenstoffatomen und bis zu 5 gleichen oder verschiedenen Halogenatomen, insbeondere Fluor- oder Chloratome, wobei Trifluormethyl beispielhaft genannt sei, ferner Alkoxy, Alkylthio und Alkylsulfonyl mit jeweils 1 bis 4 Kohlenstoffatomen, weiterhin Aminosulfonyl, Cyano und Nitro sowie gegebenenfalls durch Chlor substituiertes Phenyl oder Phenoxy. Der Index m steht vorzugsweise für 1, 2 oder 3.

Als Beispiele für besonders bevorzugt in Frage kommende Acetanilide der Formel (VI) seien die in der nachfolgenden Tabelle 2 aufgeführten Verbindungen genannt.

Tabelle 2

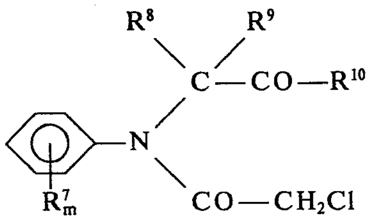

(VI)

| Bsp. Nr. | $R^7_m$ | $R^8$ | $R^9$ | $R^{10}$ |
|---|---|---|---|---|
| VI-1 | 2,6-$(CH_3)_2$ | H | H | 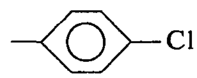 |
| VI-2 | 2-$CH_3$<br>6-$C_2H_5$ | H | H | $-C(CH_3)_3$ |
| VI-3 | 2-$CH_3$ | H | H | 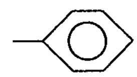 |
| VI-4 | 2-$CH_3$ | H | H | 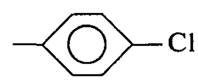 |
| VI-5 | 2,6-$(C_2H_5)_2$ | H | H | 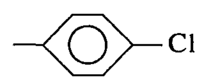 |
| VI-6 | 2,6-$(C_2H_5)_2$ | H | H | 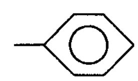 |
| VI-7 | 2-Cl | H | H | 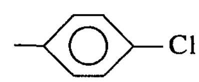 |
| VI-8 | 2,6-$(CH_3)_2$ | H | H | 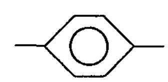 |

Fortsetzung

| Bsp. Nr. | $R^7_m$ | $R^8$ | $R^9$ | $R^{10}$ |
|---|---|---|---|---|
| VI-9 | 4-Cl | H | H | 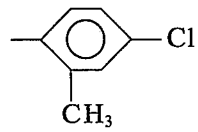 |
| VI-10 | 2,6-$(CH_3)_2$ | $CH_3$ | H | |
| VI-11 | 2,6-(i-$C_3H_7)_2$ | H | H | 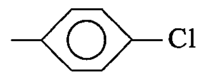 |
| VI-12 | 2,6-$(C_2H_5)_2$<br>4-$CH_3$ | H | H | 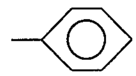 |
| VI-13 | 2,6-(i-$C_3H_7)_2$ | H | H | 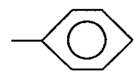 |
| VI-14 | 2,6-$(CH_3)_2$ | H | H | 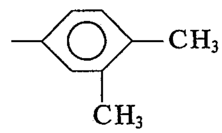 |
| VI-15 | 2-$C_2H_5$<br>5-$CH_3$ | H | H | 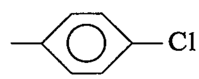 |
| VI-16 | 2,5-$(CH_3)_2$ | H | H | 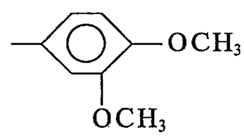 |
| VI-17 | 2-$C_2H_5$<br>4,6-$(CH_3)_2$ | H | H | 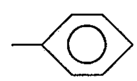 |
| VI-18 | 2,6-$(CH_3)_2$ | H | H | 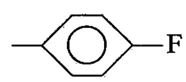 |
| VI-19 | 2,4,6-$(CH_3)_3$ | H | H | 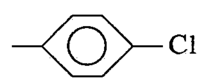 |
| VI-20 | 2,4,6-$(CH_3)_3$ | H | H | 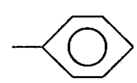 |
| VI-21 | 2,6-$(CH_3)_2$ | H | 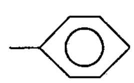 | 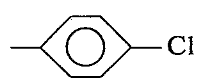 |
| VI-22 | 2,6-$(CH_3)_2$ | H | $CH_3$ | 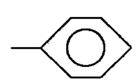 |
| VI-23 | 3,5-$(CF_3)_2$ | H | H | 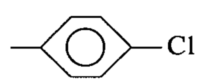 |
| VI-24 | 2,6-$(CH_3)_2$ | H | H | 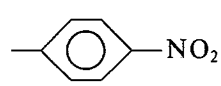 |
| VI-25 | 2,6-$(CH_3)_2$ | H | H | 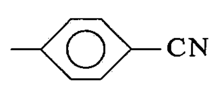 |

Fortsetzung

| Bsp. Nr. | $R^7_m$ | $R^8$ | $R^9$ | $R^{10}$ |
|---|---|---|---|---|
| VI-26 | 2,6-$(CH_3)_2$ | H | H | 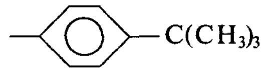 |
| VI-27 | 2,6-$(CH_3)_2$<br>4-$SC_2NH_2$ | H | H | 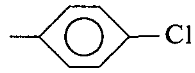 |
| VI-28 | 2-Cl<br>6-$CH_3$ | H | H | 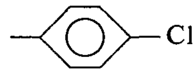 |
| VI-29 | 2-$C_2H_5$<br>6-$CH_3$ | $CH_3$ | $CH_3$ | 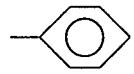 |
| VI-30 | 2-$C_2H_5$<br>6-$CH_3$ | $CH_3$ | $CH_3$ | 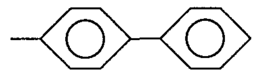 |
| VI-31 | 2-$C_2H_5$<br>6-$CH_3$ | $CH_3$ | $CH_3$ | 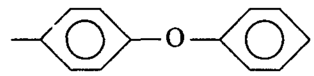 |
| VI-32 | 2-$C_2H_5$<br>6-$CH_3$ | $CH_3$ | $CH_3$ | 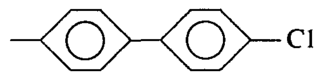 |
| VI-33 | 2-$C_2H_5$<br>6-$CH_3$ | $CH_3$ | $CH_3$ | 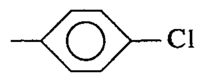 |
| VI-34 | 2-$C_2H_5$<br>6-$CH_3$ | H | $CH_3$ | 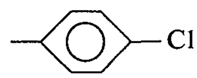 |
| VI-35 | 2-$C_2H_5$<br>6-$CH_3$ | H | $CH_3$ | 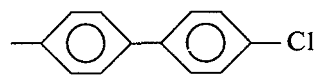 |
| VI-36 | 2,6-$(CH_3)_2$ | H | 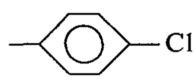 | 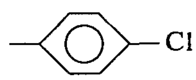 |
| VI-37 | 2,6-$(CH_3)_2$ | H | 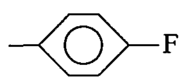 | 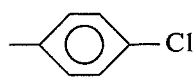 |
| VI-38 | 2,6-$(CH_3)_2$ | H | 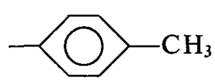 | 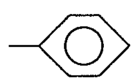 |
| VI-39 | 2,6-$(CH_3)_2$ | H | 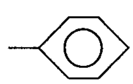 | 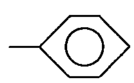 |
| VI-40 | 2,6-$(CH_3)_2$ | H | 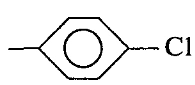 | 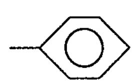 |
| VI-41 | 2,6-$(CH_3)_2$ | H | $CH_3$ | 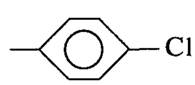 |

Die Acetanolide der Formel (VI) und deren herbizide Wirksamkeit sind bereits bekannt (vgl. DE-OS 2 726 253).

In der Formel (VII) steht A vorzugsweise für Sauerstoff, Schwefel oder die Gruppierung $-NR^{16}$, worin

$R^{16}$ vorzugsweise für Wasserstoff, geradkettiges oder verzweigtes Alkyl mit 1 bis 4 Kohlenstoffatomen sowie für Aryl mit 6 bis 10 Kohlenstoffatomen, insbesondere Phenyl, steht, wobei jeder dieser Arylreste substituiert sein kann durch Halogen, Alkyl mit 1 bis 4 Kohlenstoffatomen, Alkoxy mit 1 bis 2 Kohlenstoffatomen, Alkylthio mit 1 oder 2 Kohlenstoffatomen, Cyano, Nitro und/oder Halogenalkyl mit bis zu 2 Kohlenstoff- und bis zu 5 gleichen oder verschiedenen Halogenatomen, wobei als Halogene insbesondere Fluor und Chlor genannt seien. $R^{13}$ steht vorzugsweise für Wasserstoff oder Methyl. $R^{14}$ steht in der Formel (VII) vorzugsweise für Wasserstoff, geradkettiges oder verzweigtes Alkyl mit 1 bis 4 Kohlenstoffatomen, Halogenalkyl mit bis zu 3 Kohlenstoffatomen und bis zu 5 gleichen oder verschiedenen Halogenatomen, wobei als Halogene insbesondere Fluor und Chlor stehen, beispielhaft sei Trifluormethyl genannt, ferner vorzugsweise für Alkenyl und Alkinyl mit 2 bis 4 Kohlenstoffatomen, Cycloalkyl mit 5 bis 7 Kohlenstoffatomen sowie für Halogen, insbesondere Fluor, Chlor oder Brom. $R^{14}$ steht ferner vorzugsweise für Aryl mit 6 bis 10 Kohlenstoffatomen, insbesondere Phenyl, wobei jeder dieser Arylreste substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen, wobei als Halogene insbesondere Fluor oder Chlor stehen und Trifluormethyl als Beispiel für Halogenalkyl speziell genannt sei. $R^{14}$ steht ferner vorzugsweise für Aralkyl mit 6 bis 10 Kohlenstoffatomen im Arylteil und 1 bis 4 Kohlenstoffatomen im Alkylteil, insbesondere für Benzyl, wobei jeder dieser Aralkylreste im Arylteil substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen, wobei als Halogene insbesondere Fluor oder Chlor stehen und Trifluormethyl als Beispiel für Halogenalkyl speziell genannt sei. Außerdem steht $R^{14}$ für die Gruppierungen $-OR^{17}$, $-SR^{17}$ und $-NR^{16}R^{17}$, worin $R^{16}$ vorzugsweise für diejenigen Reste steht, die oben bereits vorzugsweise für diesen Rest genannt wurden. $R^{17}$ steht in diesen Gruppierungen für Wasserstoff, geradkettiges oder verzweigtes Alkyl mit 1 bis 4 Kohlenstoffatomen, Halogenalkyl mit 1 bis 3 Kohlenstoffatomen und bis zu 5 gleichen oder verschiedenen Halogenatomen, wobei als Halogene insbesondere Fluor und Chlor stehen, beispielhaft sei Trifluormethyl genannt, ferner vorzugsweise für Alkenyl und Alkinyl mit 2 bis 4 Kohlenstoffatomen, Cycloalkyl mit 5 bis 7 Kohlenstoffatomen sowie für Aralkyl mit 6 bis 10 Kohlenstoffatomen im Arylteil und 1 bis 4 Kohlenstoffatomen im Alkylteil, insbesondere für Benzyl, wobei jeder dieser Aralkylreste im Arylteil substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen, wobei als Halogene insbesondere Fluor oder Chlor stehen und Trifluormethyl als Beispiel für Halogenalkyl speziell genannt sei. In der Formel (VII) steht $R^{11}$ vorzugsweise für geradkettiges oder verzweigtes Alkyl mit 1 bis 4 Kohlenstoffatomen. $R^{12}$ steht in der Formel (VII) vorzugsweise für geradkettiges oder verzweigtes Alkyl mit 1 bis 4 Kohlenstoffatomen oder für die Halogene Fluor, Chlor und Brom. $R^{15}$ steht in der Formel (VII) vorzugsweise für Chlor, Brom und Jod. Der Index p steht für die Zahlen 0, 1 oder 2.

Als Beispiele für Acetanilide der Formel (VII) seien im einzelnen die in der nachfolgenden Tabelle 3 aufgeführten Verbindungen genannt:

Tabelle 3

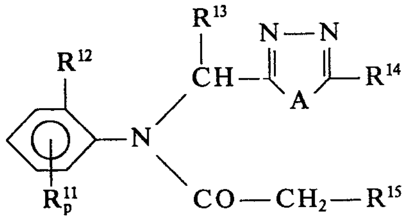

(VII)

| Bsp. Nr. | $R^{13}$ | $R^{14}$ | $R^{12}$ | $R^{11}$ | A | $R^{15}$ |
|---|---|---|---|---|---|---|
| VII-1 | H | $CH_3$ | $CH_3$ | 6-$C_2H_5$ | O | Cl |
| VII-2 | H | $SCH_3$ | $C_2H_5$ | 6-$C_2H_5$ | $N—CH_3$ | Cl |
| VII-3 | H | $CH_3$ | $C_2H_5$ | 6-$C_2H_5$ | O | Cl |
| VII-4 | H | $CH_3$ | $CH_3$ | 6-$CH_3$ | O | Cl |
| VII-5 | H | $CH_3$ | $C(CH_3)_3$ | — | O | Cl |
| VII-6 | H | $—S—CH_2—CH{=}CH_2$ | $C_2H_5$ | 6-$C_2H_5$ | 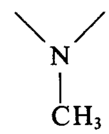 | Cl |
| VII-7 | H | $—S—CH_2—$ 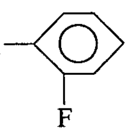 | $CH_3$ | 6-$C_3H_5$ | 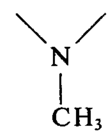 | Cl |
| VII-8 | H | $C_2H_5$ | $CH_3$ | 6-$C_2H_5$ | O | Cl |
| VII-9 | H | $C_2H_5$ | $C_2H_5$ | 6-$C_2H_5$ | O | Cl |
| VII-10 | H | i-$C_3H_7$ | $CH_3$ | 6-$C_2H_5$ | O | Cl |
| VII-11 | H | $CH_3$ | $CH_3$ | 3-$CH_3$ | 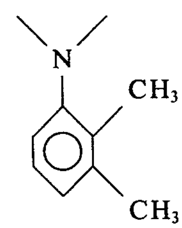 | Cl |
| VII-12 | H | $CH_3$ | $C_2H_5$ | 6-$C_2H_5$ | O | Br |
| VII-13 | H | $CH_3$ | $CH_3$ | 6-$C_2H_5$ | O | Br |
| VII-14 | H | $CH_3$ | i-$C_3H_7$ | 6-i-$C_3H_7$ | O | Cl |

Die Acetanilide der Formel (VII) und deren herbizide Wirksamkeit sind bereits bekannt (vgl. EP-OS 3 539).

Weitere bevorzugt in Frage kommende Acetanilide, bei denen die Verbindungen der Formel (I) als Gegenmittel eingesetzt werden können, sind die Verbindungen der Formeln (VIII) und (IX). Diese Stoffe und deren herbizide Wirksamkeit sind bereits bekannt (vgl. US-PS 3 442 945 und DE-OS 2 338 340).

Die erfindungsgemäß verwendbaren N,N'-Bis-(halogenacyl)-diaza-cycloalkane der Formel (I) eignen sich insbesondere zum Schutz von wichtigen Kulturpflanzen, wie Mais, Sojabohnen, Baumwolle, Zukkerrüben, Getreide, Reis und Zuckerrohre vor Herbizidschädigungen durch Acetanilide.

Die erfindungsgemäßen Wirkstoffkombinationen bestehend aus einem N,N'-Bis-(halogenacyl)-diaza-cycloalkan der Formel (I) und mindestens einem herbizid wirksamen Acetanilid zeigen eine sehr gute Wirkung gegen Unkräuter und Ungräser in zahlreichen Nutzpflanzenkulturen. Sie können daher zur selektiven Unkrautbekämpfung in zahlreichen Nutzpflanzenkulturen verwendet werden. – Unter

Unkräuter im weitesten Sinne sind hierbei alle Pflanzen zu verstehen, die an Orten wachsen, wo sie unerwünscht sind.

Die erfindungsgemäßen Wirkstoffkombinationen können zum Beispiel bei folgenden Pflanzen angewendet werden.

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Rorippa, Rotala, Lindernia, Lamium, Veronica, Abutilon, Emex, Datura, Viola, Galeopsis, Papaver, Centaurea.

Dicotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleotharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Insbesondere eignen sich die erfindungsgemäßen Wirkstoffkombinationen zur selektiven Unkrautbekämpfung in Mais, Sojabohnen, Baumwolle, Zuckerrüben, Getreide, Reis und Zuckerrohr.

Die erfindungsgemäß verwendbaren Gegenmittel können gegebenenfalls im Gemisch mit den herbiziden Wirkstoffen, bei denen sie eingesetzt werden, in die üblichen Formulierungen übergeführt werden, wie Lösungen, Emulsionen, Spritzpulver, Suspensionen, Pulver, Stäubemittel, Schäume, Pasten, lösliche Pulver, Granulate, Suspensions-Emulsionskonzentrate, wirkstoffimprägnierte Natur- und synthetische Stoffe, Feinstverkapselungen in polymeren Stoffen und in Hüllmassen für Saatgut.

Diese Formulierungen werden in bekannter Weise hergestellt, zum Beispiel durch Vermischen der erfindungsgemäß verwendbaren Gegenmittel gegebenenfalls im Gemisch mit den herbiziden Wirkstoffen, bei denen sie eingesetzt werden, mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid sowie Wasser; natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Mamor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel: nichtionogene und anionische Emulgatoren, wie Polyoxyethylen-Fettsäure-Ester, Poly-ethylen-Fettalkohol-Ether, z. B. Alkylaryl-polyglykol-ether, Alkylsulfonate, Alkylsulfate, Arylsulfonate sowie Eiweißhydrolysate; als Dispergiermittel: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie

Salze von Eisen, Mangan, Bor, Kupfer, Kobalt, Molybdän und Zink verwendet werden.

Die Formulierungen enthalten im allgemeinen zwischen 0,1 und 95 Gew.-% an Gegenmittel bzw. an Gegenmittel und herbizidem Wirkstoff, vorzugsweise zwischen 0,5 und 90%.

Die erfindungsgemäß verwendbaren Gegenmittel können als solche oder in ihren Formulierungen auch in Mischung mit herbiziden Wirkstoffen eingesetzt werden, wobei Fertigformulierung oder Tankmischung möglich ist. Auch eine Mischung mit anderen bekannten Wirkstoffen wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Daß erfindungsgemäß verwendbare Gegenmittel bzw. Gemische aus erfindungsgemäß verwendbaren Gegenmitteln und herbizidem Wirkstoff können als solche, in Form ihrer Formulierungen oder den daraus durch weiteres Verdünnen bereiteten Anwendungsformen, wie gebrauchsfertige Lösungen, Suspensionen, Emulsionen, Pulver und Granulate angewandt werden. Die Anwendung geschieht in üblicher Weise, z. B. durch Gießen, Spritzen, Sprühen, Stäuben oder Streuen, Trockenbeizen, Feuchtbeizen, Naßbeizen, Schlämmbeizen oder Inkrustieren.

Die erfindungsgemäßen Gegenmittel können nach den für derartige Antidote üblichen Methoden ausgebracht werden. So können die erfindungsgemäßen Gegenmittel entweder vor oder nach dem Herbizid ausgebracht oder zusammen mit dem Herbizid appliziert werden. Ferner können Kulturpflanzen durch Saatgutbehandlung mit den Gegenmitteln vor der Saat (Beizung) vor Schäden geschützt werden, wenn das Herbizid vor oder nach der Saat angewendet wird. Eine weitere Einsatzmöglichkeit besteht darin, daß man die Gegenmittel bei der Aussaat in die Saatfurche ausbringt. Wenn es sich bei den Pflanzen um Stecklinge handelt, so können diese vor der Auspflanzung mit den Gegenmitteln behandelt werden.

Beim Einsatz der erfindungsgemäßen Gegenmittel kommen die ortsüblichen Aufwandmengen an den jeweiligen Herbiziden zur Anwendung. Die Aufwandmengen an herbizidem Wirkstoff schwanken zwischen 0,5 und 5 kg/ha. Die Aufwandmenge an Gegenmittel ist unabhängig vom Herbizid und der Aufwandmenge des herbiziden Wirkstoffes. Im allgemeinen liegen die Aufwandmengen an erfindungsgemäßen Gegenmitteln bei Flächenbehandlung zwischen 0,1 und 5 kg/ha, vorzugsweise zwischen 0,2 und 4 kg/ha. Bei der Saatgutbehandlung liegen die Aufwendungen an erfindungsgemäßen Gegenmitteln im allgemeinen zwischen 10 und 300 g pro Kilogramm Saatgut, vorzugsweise zwischen 25 und 200 g pro Kilogramm Saatgut.

In den erfindungsgemäßen Wirkstoffkombinationen können die Gewichtsverhältnisse von Gegenmitteln zu herbiziden Wirkstoffen in relativ großen Bereichen schwanken. Im allgemeinen entfallen auf 1 Gewichtsteil an herbizidem Wirkstoff 0,05 bis 1,0 Gewichtsteile, vorzugsweise 0,1 bis 0,5 Gew.-Teile an Gegenmittel der Formel (I).

Die gute Wirksamkeit der erfindungsgemäßen Gegenmittel geht aus dem nachfolgendem Beispiel hervor.

## Beispiel A

### Pre-emergence-Test

Lösungsmittel:
5 Gew.-Teile Aceton
1 Gew.-Teil Alkylarylpolyglykolether mit durchschnittlich 18 Oxyethylengruppen pro Molekül

Zur Herstellung einer zweckmäßigen Wirkstoffzubereitung vermischt man 1 Gew.-Teil herbiziden Wirkstoff bzw. Gegenmittel bzw. eines Gemisches aus Gegenmittel und herbizidem Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Herbizid-Zubereitung bzw. mit der Gegenmittel-Zubereitung bzw. mit der Zubereitung aus Gegenmittel und herbizidem Wirkstoff begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

Wirkstoffe, Aufwandmengen und Resultate gehen aus der nachfolgenden Tabelle hervor:

Tabelle A
Pre-emergence-Test

| Wirkstoff Herbizid | Wirkstoff-aufwand an Herbizid kg/ha | Wirkstoff Gegenmittel | Wirkstoff-aufwand an Gegenmittel kg/ha | Mais | Schädigung bei Echinochloa % | Amaranthus |
|---|---|---|---|---|---|---|
| | — | 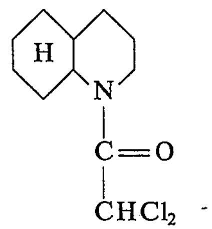 (bekannt) | 3 | 0 | 0 | 0 |
| | — | 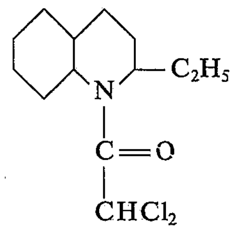 (bekannt) | 3 | 0 | 0 | 0 |
| 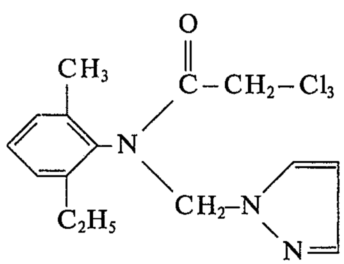 | | — | — | 90 | 100 | 100 |

Fortsetzung

| Wirkstoff Herbizid | Wirkstoff-aufwand an Herbizid kg/ha | Wirkstoff Gegenmittel | Wirkstoff-aufwand an Gegenmittel kg/ha | Mais | Schädigung bei Echinochloa % | Amaranthus |
|---|---|---|---|---|---|---|
| 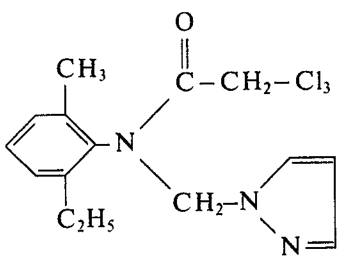 | | 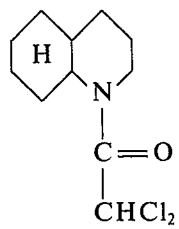 (bekannt) | 3 | 80 | 100 | 100 |
| 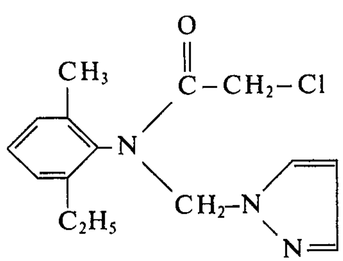 | 3 | 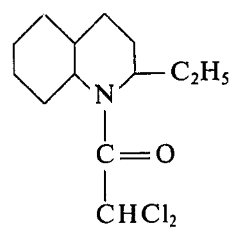 (bekannt) | 3 | 80 | 100 | 100 |
| — | — | 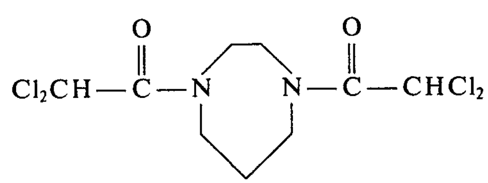 (erfindungsgemäß) | 3 | 0 | 0 | 0 |

Fortsetzung

| Wirkstoff Herbizid | Wirkstoffaufwand an Herbizid kg/ha | Wirkstoff Gegenmittel | Wirkstoffaufwand an Gegenmittel kg/ha | Mais | Schädigung bei Echinochloa % | Amaranthus |
|---|---|---|---|---|---|---|
| — | — | 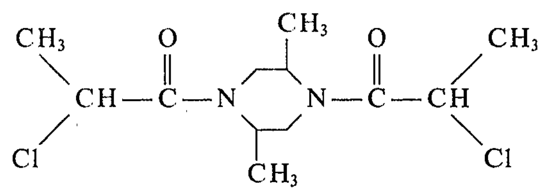 (erfindungsgemäß) | 3 | 0 | 0 | 0 |
| 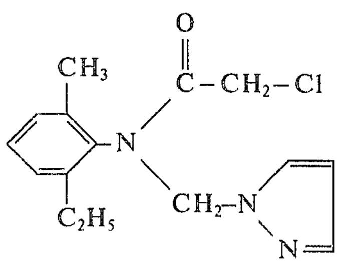 | 3 | 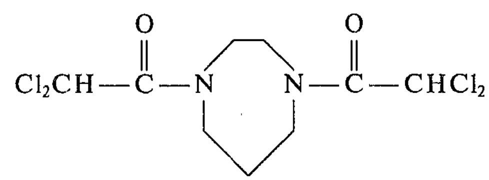 (erfindungsgemäß) | 1 | 0 | 100 | 100 |
| 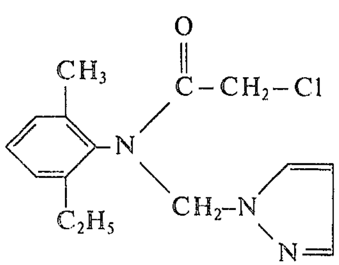 | 3 | 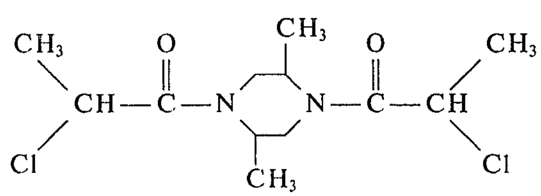 (erfindungsgemäß) | 1 | 0 | 100 | 100 |

## Herstellungsbeispiele

### Beispiel 1

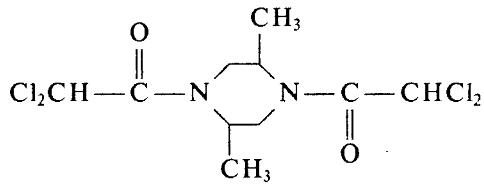

a) Zu einer Lösung von 22,8 g (0,2 Mol) 2,5-Dimethylpiperazin in 150 ml Toluol werden bei 20°C unter Rühren 29,4 g (0,2 Mol) Dichloracetylchlorid getropft. Es wird 5 Stunden bei 20°C nachgerührt. Danach wird der entstandene Niederschlag abgesaugt und zweimal aus Toluol umkristallisiert. Man erhält auf diese Weise 8 g (24 % der Theorie) an N,N'-Bis-(dichloracetyl)-2,5-dimethylpiperazin in Form farbloser Kristalle vom Schmelzpunkt 215°C.

b) Zu einer Lösung von 22,8 g (0,2 Mol) 2,5-Dimethylpiperazin in 150 ml Acetonitril werden bei 20°C unter Rühren 29,4 g (0,2 Mol) Dichloracetylchlorid getropt. Man läßt 5 Stunden bei 20°C nachreagieren, gießt dann das Reaktionsgemisch in 300 ml Wasser, saugt den anfallenden Niederschlag ab und kristallisiert ihn aus Toluol um. Man erhält auf diese Weise 12 g (36 % der Theorie) an N,N'-Bis-(dichloracetyl)-2,5-dimethyl-piperazin in Form farbloser Kristalle vom Schmelzpunkt 214°C.

In analoger Weise werden die in der nachfolgenden Tabelle 4 aufgeführten Verbindungen der Formel (I) hergestellt:

Tabelle 4

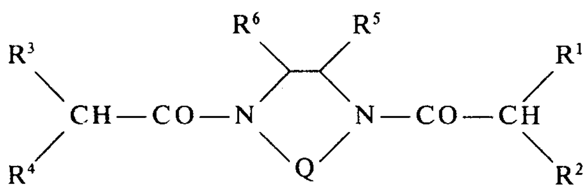

(I)

| Bsp.-Nr. | $R^1$ | $R^2$ | $R^3$ | $R^4$ | $R^5$ | $R^6$ | Q | Schmelzpunkt °C |
|---|---|---|---|---|---|---|---|---|
| 2 | $CH_3$ | Cl | $CH_3$ | Cl | H | $CH_3$ | $-CH_2-CH-$<br>$CH_3$ | 167 |
| 3 | Cl | Cl | Cl | Cl | H | H | $-(CH_2)_3-$ | 130 |
| 4 | $CH_3$ | Cl | $CH_3$ | Cl | H | H | $-(CH_2)_3-$ | teilkristallin |
| 5 | Cl | Cl | Cl | Cl | H | H | $-CH_2-CH_2-$ | 217 |
| 6 | $CH_3$ | Cl | $CH_3$ | Cl | H | H | $-CH_2-CH_2-$ | |
| 7 | Cl | Cl | Cl | Cl | $CH_3$ | H | $-CH_2-CH-$<br>$CH_3$ | 225 |
| 8 | Cl | $CH_3$ | Cl | $CH_3$ | $CH_3$ | H | $-CH_2-CH-$<br>$CH_3$ | 137 |
| 9 | Cl | Cl | Cl | Cl | $CH_3$ | H | $-CH_2-CH_2-$ | 160 |

**Patentanprüche**

1. Verwendung von N,N'-Bis-(halogenacyl)-diaza-cycloalkanen der Formel

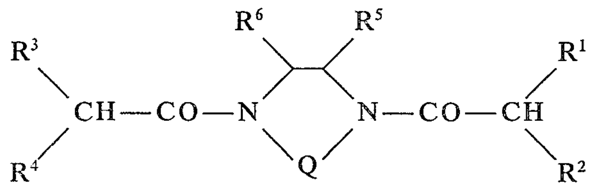

(I)

in welcher

$R^1$ für geradkettiges oder verzweigtes Alkyl mit 1 bis 4 Kohlenstoffatomen, Chlor oder Brom steht,
$R^2$ für Chlor oder Brom steht,
$R^3$ für geradkettiges oder verzweigtes Alkyl mit 1 bis 4 Kohlenstoffatomen, Chlor oder Brom steht,
$R^4$ für Chlor oder Brom steht,
$R^5$ für Wasserstoff oder Alkyl mit 1 oder 2 Kohlenstoffatomen steht,
$R^6$ für Wasserstoff oder Alkyl mit 1 oder 2 Kohlenstoffatomen steht und
Q für eine gegebenenfalls ein- oder mehrfach durch Methyl oder Ethyl substituierte Alkylenkette mit 2 oder 3 Kohlenstoffatomen in der Alkylenkette steht,

zum Schutz von Kulturpflanzen vor Schädigungen durch herbizid wirksame Acetanilide.

2. Verwendung gemäß Anspruch 1, dadurch gekennzeichnet, daß man als N,N'-Bis-(halogenacyl)-diaza-cycloalkan die Verbindung der Formel

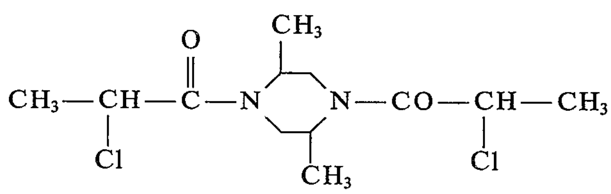

einsetzt.

3. Verwendung gemäß Anspruch 1, dadurch gekennzeichnet, daß man als N,N'-Bis-(halogenacyl)-diaza-cycloalkan die Verbindung der Formel

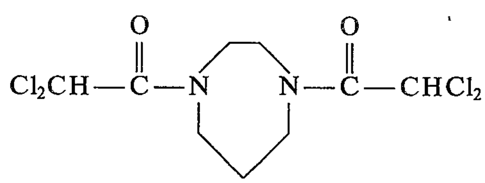

einsetzt.

4. Mittel zur selektiven Unkrautbekämpfung in Nutzpflanzenkulturen, gekennzeichnet durch einen Gehalt an einer Wirkstoffkombination bestehend aus mindestens einem N,N'-Bis-(halogenacyl)-diaza-cycloalkan der Formel (I) gemäß Anspruch 1 und mindestens einem herbizid wirksamen Acetanilid.

5. Verwendung von Wirkstoffkombinationen gemäß Anspruch 4 zur selektiven Unkrautbekämpfung in Nutzpflanzenkulturen.

**Claims**

1. Use of N,N'-bis-(halogenoacyl)-diaza-cycloalkanes of the formula

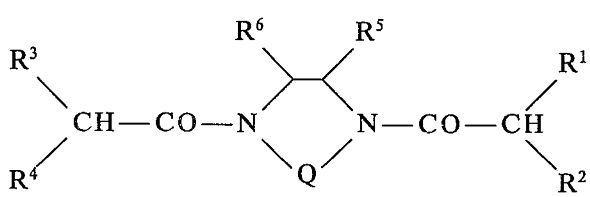

(I)

in which

$R^1$ represents straight-chain or branched alkyl having 1 to 4 carbon atoms, chlorine or bromine,
$R^2$ represents chlorine or bromine,
$R^3$ represents straight-chain or branched alkyl having 1 to 4 carbon atoms, chlorine or bromine,
$R^4$ represents chlorine or bromine,
$R^5$ represents hydrogen or alkyl having 1 or 2 carbon atoms,
$R^6$ represents hydrogen or alkyl having 1 or 2 carbon atoms and
Q represents an alkylene chain which has 2 or 3 carbon atoms in the alkylene chain and is optionally monosubstituted or polysubstituted by methyl or ethyl

for protecting crop plants from damage by herbicidally active acetanilides.

2. Use according to Claim 1, characterised in that the N,N'-bis-(halogenoacyl)-diaza-cycloalkane employed is the compound of the formula

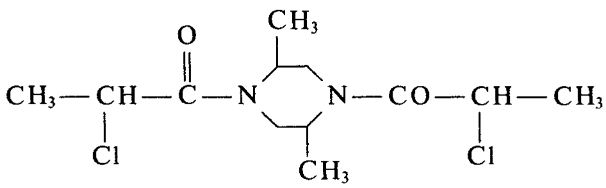

3. Use according to Claim 1, characterised in that the N,N'-bis-(halogenoacyl)-diaza-cycloalkane employed is the compound of the formula

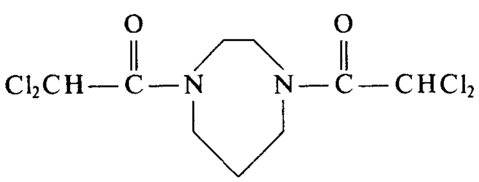

4. Agents for selectively combating weeds in crops of useful plants, characterised in that they contain an active compound combination consisting of at least one N,N'-bis-(halogenoacyl)-diaza-cycloalkane of the formula (1) according to Claim 1 and at least one herbicidally active acetanilide.

5. Use of active compound combinations according to Claim 4 for selectively combating weeds in crops of useful plants.

## Revendications

1. Utilisation des N,N'-bis-(halogénoacyl)-diaza-cycloalcanes de formule

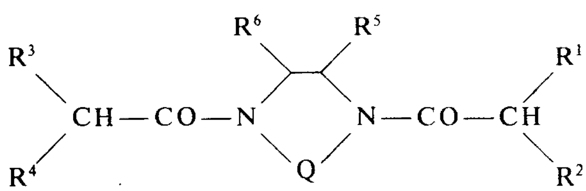

(I)

dans laquelle

$R^1$ représente un groupe alkyle à chaîne droite ou ramifiée contenant de 1 à 4 atomes de carbone, le chlore ou le brome,
$R^2$ représente le chlore ou le brome,
$R^3$ représente un groupe alkyle à chaîne droite ou ramifiée contenant de 1 à 4 atomes de carbone, le chlore ou le brome,
$R^4$ représente le chlore ou le brome,
$R^5$ représente l'hydrogène ou un groupe alkyle contenant 1 ou 2 atomes de carbone,
$R^6$ représente l'hydrogène ou un groupe alkyle contenant 1 ou 2 atomes de carbone et
Q représente une chaîne alkylène éventuellement mono- ou polysubstituée par des groupes méthyle ou éthyle et contenant 2 ou 3 atomes de carbone dans la chaîne alkylene,

pour la protection des plantes cultivées contre les dommages provoqués par les acétanilides à activité herbicide.

2. Utilisation selon la revendication 1, caractérisée en ce que l'on utilise en tant que N,N'-bis-(halogénoacyl)-diaza-cycloalcane le composé de formule

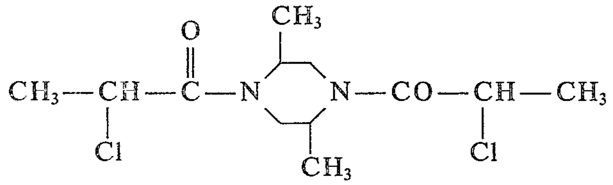

3. Utilisation selon la revendikation 1, caractérisée en ce que l'on utilise en tant que N,N'-bis-(halogénoacyl)-diaza-cycloalcane le composé de formule

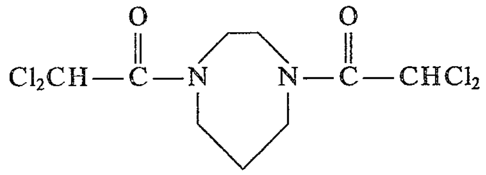

4. Produit pour combattre sélectivement les mauvaises herbes dans les cultures de plantes utiles, caractérisé en ce qu'il contient une combinaison de substances actives consistant en au moins un N,N'-bis-(halogénoacyl)-diaza-cycloalcane de formule I selon la revendication 1, et au moins un acétanilide à activité herbicide.

5. Utilisation des combinaisons de substances actives selon la revendication 4, dans la lutte sélective contre les mauvaises herbes dans les cultures de végétaux utiles.